# EUROPEAN PATENT SPECIFICATION

(11) **EP 4 445 960 B1**
(45) Date of publication and mention of the grant of the patent: **18.11.2026**
(21) Application number: 22904111.6
(22) Date of filing: 30.11.2022
(51) Int. Cl.: A61Q 1/00, A61Q 1/06, A61Q 1/10, A61Q 1/12, A61K 8/81, A61K 8/891

(54) **OIL-GELLING AGENT AND COSMETIC**
ÖLGELIERMITTEL UND KOSMETIKUM
AGENT DE GÉLIFICATION D'HUILE ET PRODUIT COSMÉTIQUE

(30) Priority: 07.12.2021 JP 2021198472
(43) Date of publication of application: 16.10.2024
(73) Proprietor: SHIN-ETSU CHEMICAL CO., LTD., Tokyo 1000005 (JP)
(72) Inventor: HATA Ryunosuke, Annaka-shi, Gunma 379-0224 (JP)
(74) Representative: Mewburn Ellis LLP
(86) International application number: PCT/JP2022/044196
(87) International publication number: WO 2023/106180

(56) References cited:
- WO-A1-2016/098456
- JP-A- 2012 072 081
- JP-A- 2016 503 792
- US-A1- 2021 212 919

## Description

### TECHNICAL FIELD

The present invention relates to an oil-gelling agent and a cosmetic containing the oil-gelling agent.

### BACKGROUND ART

Oil-gelling agents capable of gelling hydrophobic oils and silicone oils are widely used as ingredients for cosmetics and other products owing to their ability to solidify oils. Hydrocarbon polymers, such as polyethylene waxes, long-chain fatty acids, and long-chain alcohols are well known as such gelling agents. Furthermore, oil-gelling agents having a characteristic structure, specifically, being based on a sugar backbone in which the hydroxyl groups are partly acylated are known and frequently used in cosmetics (Patent Document 1).

In many cases, these oil-gelling agents gel oils by utilizing hydrophobic interactions and hydrogen bonding (or dipole-dipole interactions). Thus, a gelling agent with a strong hydrophobic interaction sometimes encounters difficulties in gelling a hydrophobic oil, and conversely the gelation of an oil with hydrogen bonding properties is sometimes difficult with a gelling agent having strong hydrogen bonding properties. Against these backgrounds, there is a demand for a gelling agent that can gel various kinds of oils.

It is known that a gelling agent based on an acrylic polymer can be controlled in hydrophobicity and hydrogen bonding properties by control of the ratio of constituent monomers (Patent Document 2). However, there is room for improvement with respect to the variety of oils that can be gelled.

### PRIOR ART DOCUMENTS

### PATENT DOCUMENTS

Patent Document 1: JP-A 2005-145851
Patent Document 2: WO 2016/098456

### SUMMARY OF INVENTION

### TECHNICAL PROBLEM

The present invention has been made in view of the circumstances discussed above. It is therefore an object of the present invention to provide an oil-gelling agent capable of gelling various kinds of oils, such as hydrocarbon oils and higher alcohols.

### SOLUTION TO PROBLEM

As a result of intensive studies directed to achieving the above object, the present inventor has found that a gelling agent can gel various kinds of oils, such as hydrocarbon oils and higher alcohols, when the gelling agent is designed in such a manner that an amphiphilic (meth)acrylic monomer is used as principal constituent units and the polarity for an oil to be gelled is optimized by the incorporation of a hydrophilic (meth)acrylic monomer. The present invention has been completed based on the finding.

Specifically, the present invention provides an oil-gelling agent and a cosmetic described below.
1. An oil-gelling agent including a copolymer including constituent units from:
   an amphiphilic (meth)acrylic monomer (a) represented by general formula (1) below: 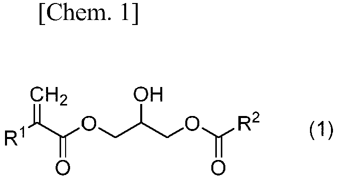 wherein R¹ is a hydrogen atom or a methyl group and R² is a linear or branched C₁₋₂₁ alkyl group; and
   a hydrophilic (meth)acrylic monomer (b) represented by general formula (2) below: 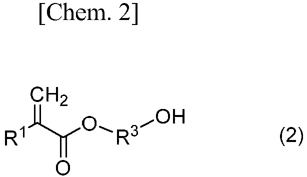 wherein R¹ is a hydrogen atom or a methyl group and R³ is a linear or branched C₂₋₁₀ alkylene group,
   the mass ratio (b)/(a) of (b) to (a) being 0 < [(b)/(a)] ≤ 0.2.
2. The oil-gelling agent described in 1, further including constituent units from 35 to 50 wt% of a monomer (c) based on all the monomers, the monomer (c) being a silicone macromer represented by general formula (3) below: 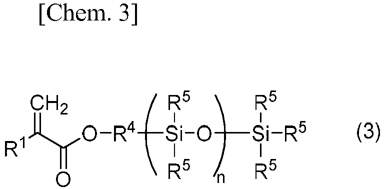 wherein R¹ is a hydrogen atom or a methyl group, R⁴ is a divalent organic group, R⁵ independently at each occurrence is a group selected from a hydrogen atom, a C₁₋₁₀ alkyl group, and a C₆₋₂₂ aryl group, and n is 1 to 100.
3. A cosmetic containing the oil-gelling agent described in 1 or 2.

### ADVANTAGEOUS EFFECTS OF INVENTION

Various kinds of oils, such as hydrocarbon oils and higher alcohols, can be gelled by the use of the oil-gelling agent of the present invention.

### DESCRIPTION OF EMBODIMENTS

The present invention will be described in detail below.

An oil-gelling agent of the present invention is a copolymer that is obtained by copolymerizing a specific amphiphilic (meth)acrylic monomer (a) and a specific hydrophilic (meth)acrylic monomer (b) and includes these monomers as constituent units. In the following, R¹ and the like that are used in a plurality of formulas are independent at each occurrence.

### [Amphiphilic (meth)acrylic monomers (a)]

The amphiphilic (meth)acrylic monomer (a) is a (meth)acrylic monomer represented by general formula (1) below. Such monomers may be used singly, or two or more may be used in combination. 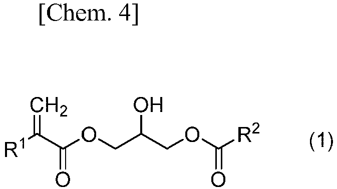 wherein R¹ is a hydrogen atom or a methyl group and R² is a linear or branched C₁₋₂₁ alkyl group.

R¹ is a hydrogen atom or a methyl group and R² is a linear or branched C₁₋₂₁ alkyl group. From the point of view of hydrophobic interactions, R² is preferably a C₁₁₋₂₁ alkyl group, and more preferably a C₁₅₋₂₁ alkyl group.

### [Methods for synthesizing the amphiphilic (meth)acrylic monomers]

The amphiphilic (meth)acrylic monomer may be obtained by reacting glycidyl methacrylate or glycidyl acrylate with a fatty acid. From the point of view of the reaction rate, it is preferable to perform the reaction using a catalyst. Examples of the catalysts that may be used include quaternary alkylammonium salts, such as tetrabutylammonium bromide. To ensure that the reaction substrates and the catalyst will be mixed uniformly, it is preferable to perform the reaction in a solvent. Examples of the solvents that may be used include aprotic polar solvents, such as dimethylacetamide, dimethylformamide, and acetonitrile, and aromatic hydrocarbon solvents, such as toluene. Furthermore, the reaction is preferably followed by water-washing to increase the purity of the product.

The amphiphilic (meth)acrylic monomer represented by formula (1) may include an amphiphilic (meth)acrylic monomer represented by formula (1') below: 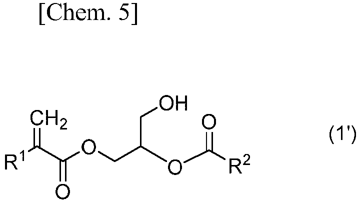 wherein R¹ and R² are the same as defined above.

The content of (1') per mol of the amphiphilic (meth)acrylic monomer of (1) is preferably 1 mol or less, more preferably 0.5 to 0 mol, and still more preferably substantially nil. The phrase "substantially nil" means that even when the monomer is present, the content is below the detection limit.

### [Hydrophilic (meth)acrylic monomers (b)]

The hydrophilic (meth)acrylic monomer (b) is a hydrophilic (meth)acrylic monomer represented by general formula (2) below: 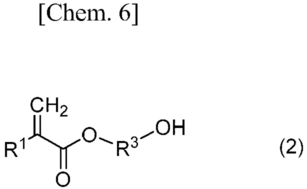 wherein R¹ is a hydrogen atom or a methyl group and R³ is a linear or branched C₂₋₁₀ alkylene group.

R¹ is a hydrogen atom or a methyl group and R³ is a linear or branched C₂₋₁₀ alkylene group. From the point of view of hydrophilicity, the number of carbon atoms in R³ is preferably 2 to 5, and more preferably 2 to 3.

The mass ratio (b)/(a) of (b) to (a) is 0 < [(b)/(a)] ≤ 0.2, preferably 0.03 ≤ [(b)/(a)] ≤ 0.2, more preferably 0.05 ≤ [(b)/(a)] ≤ 0.20, still more preferably 0.07 ≤ [(b)/(a)] ≤ 0.20, and particularly preferably 0.14 ≤ [(b)/(a)] ≤ 0.20. The above ratio ensures that the oil-gelling agent has a good balance between hydrophobicity and hydrogen bonding properties and can gel various kinds of oils.

### [Silicone macromers (c)]

The copolymer of the present invention may have constituent units from a monomer that is a silicone macromer. The grafting of silicone chains enhances the compatibility with silicone oils and further facilitates the gelation of silicone oils. Examples of the silicone macromers include (meth)acrylic-modified silicone macromers represented by general formula (3) below: 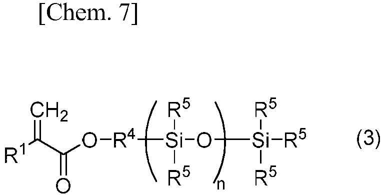 wherein R¹ is a hydrogen atom or a methyl group, R⁴ is a divalent organic group, R⁵ independently at each occurrence is a group selected from a hydrogen atom, a C₁₋₁₀ alkyl group, and a C₆₋₂₂ aryl group, and n is 1 to 100.

R¹ is a hydrogen atom or a methyl group; R⁴ is a divalent organic group, preferably a C₂₋₆ alkylene group, and more preferably a C₂₋₄ alkylene group; R⁵ independently at each occurrence is a group selected from a hydrogen atom, a C₁₋₁₀ alkyl group, and a C₆₋₂₂ aryl group, preferably a C₁₋₃ alkyl group, and more preferably a methyl group; and n is 1 to 100, preferably 10 to 90, more preferably 15 to 80, and still more preferably 20 to 70.

Examples of the compounds represented by general formula (3) include, but are not limited to, the following. 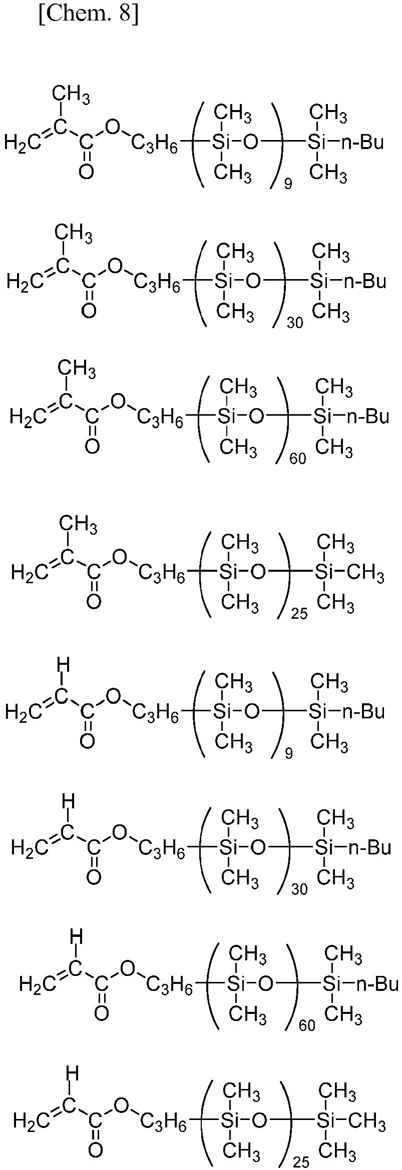 wherein n-Bu denotes an n-butyl group.

When the copolymer includes constituent units from the monomer (c), the amount of the monomer (c) based on all the monomers is preferably 35 to 50 wt%, and preferably 35 to 45 wt%. The above ratio ensures that the copolymer can well maintain a function as a gelling agent while attaining enhancements in compatibility with silicone oils.

The copolymer of the present invention may be composed solely of the monomers (a) and (b) or the monomers (a) to (c). The copolymer of the present invention may further include additional monomers other than those described above while ensuring that the advantageous effects of the present invention will not be impaired. When the copolymer includes constituent units from such an additional monomer, the amount thereof is preferably 0.05 to 5 wt% based on all the monomers. When the monomer (c) is used, the total amount of the monomer (a), the monomer (b), and the monomer (c) is preferably 95 to 99.95 wt%, more preferably 97 to 99.93 wt%, and still more preferably 98.5 to 99.9 wt% based on all the monomers; the upper limit may be 100.0 wt%. In the present invention, the phrase "based on all the monomers" means "based on all the monomers constituting the copolymer".

### [Methods for producing the copolymers]

The copolymer of the present invention is preferably obtained by polymerization in the presence of a radical polymerization initiator, such as benzoyl peroxide, lauroyl peroxide, or azobisisobutyronitrile. The polymerization method may be any of solution polymerization, emulsion polymerization, suspension polymerization, and bulk polymerization. Among these methods, solution polymerization is preferable because the molecular weight of the polymer that is obtained is easily controlled to a desired range. The polymerization reaction may be carried out in a solvent. Examples of the solvents that may be used here include aliphatic hydrocarbon organic solvents, such as pentane, hexane, decane, dodecane, hexadecane, and octadecane; aromatic hydrocarbon organic solvents, such as benzene, toluene, and xylene; alcohol organic solvents, such as methanol, ethanol, isopropyl alcohol, propanol, butanol, hexanol, and decanol; halogenated hydrocarbon organic solvents, such as chloroform and carbon tetrachloride; and ketone organic solvents, such as acetone and methyl ethyl ketone. From the point of view of the fact that the copolymer is used in cosmetic applications, it is preferable that the polymerization reaction be performed without a solvent or in ethanol or isopropanol. The copolymer may be a random, block, or graft copolymer.

In the polymer produced as described above, the polystyrene-equivalent number average molecular weight (Mn) determined by GPC is preferably 5,000 to 200,000, more preferably 7,500 to 10,000, and still more preferably 10,000 to 50,000. This range of molecular weight ensures that the copolymer can well maintain a function as a gelling agent while attaining higher solubility with respect to oils. In the present invention, the number average molecular weight is a value obtained by GPC (gel permeation chromatography) analysis using polystyrene standards under the following conditions.

### [Measurement conditions]

| | |
|---|---|
| Developing solvent: | Tetrahydrofuran (THF) |
| Flow rate: | 0.6 mL/min |
| Detector: | Differential refractive index detector (RI) |
| Columns: | TSK Guardcolumn SuperH-H |
| | TSKgel SuperHM-N (one column 6.0 mm in I.D. × 15 cm) |
| | TSKgel SuperH2500 (one column 6.0 mm in I.D. × 15 cm) |
| Apparatus: | HLC 8320 GPC (all manufactured by TOSOH CORPORATION) |
| Column temperature: | 40°C |
| Sample injection volume: | 50 µL (0.3 wt% THF solution) |

### [Oil-gelling agents]

In the present invention, the copolymer described above is used as an oil-gelling agent. The oils that are gelled are not particularly limited, and examples thereof include those described later as oil ingredients (B), with liquid oil ingredients being preferable. In particular, hydrocarbon oils, higher alcohols, and silicone oils are preferable. The oil-gelling agent is preferably capable of gelling hydrocarbon oils and higher alcohols, and is more preferably capable of gelling hydrocarbon oils, higher alcohols, and silicone oils.

### [Cosmetics]

The oil-gelling agent (A) of the present invention may be used in various kinds of cosmetic products and is particularly suited for use in lip cosmetics. The oil-gelling agent forms a gel with high temperature stability, and the resulting composition is stable and is more comfortable to use. The amount of the oil-gelling agent contained in the cosmetic of the present invention is variable depending on the form of the cosmetic. The oil-gelling agent may be used in the range of 0.5 to 99.0 wt%, preferably 1.0 to 50 wt%, relative to the whole cosmetic.

In addition to the oil-gelling agent (A), the cosmetic of the present invention may include appropriate amounts of ingredients that are commonly added to cosmetics. For example, such ingredients may be oil ingredients (B), ultraviolet-absorbing ingredients (C), water (D), surfactants (E), powders (F), compounds (G) having an alcoholic hydroxyl group in the molecular structure, water-soluble or water-swellable polymeric compounds (H), compositions (I) of a hydrophilic group-free crosslinked organopolysiloxane polymer and a liquid oil, compositions (J) of a hydrophilic group-containing crosslinked organopolysiloxane polymer and a liquid oil, silicone resins (K), and/or silicone waxes (L). These ingredients are individually described below. The compound names given below are sometimes those cited in *Kesho̅hin Hyo̅ji Meisho̅* [Japanese Cosmetic Labeling Names] or the International Nomenclature of Cosmetic Ingredients (INCI). In this Specification, when the name of an ingredient differs in these two lists, the *Kesho̅hin Hyo̅ji Meisho̅* name is given first in lower case letters, followed by, in parenthesis, the capitalized INCI name. When the name from the two lists is the same, it is followed simply by "(INCI)."

### Oil Ingredients (B)

As described above, the cosmetic of the present invention may include one, or two or more kinds of oil ingredients. Any solid, semisolid, or liquid oils that are used in common cosmetics may be used as the oil ingredients.

For example, the liquid oils may be one, or two or more kinds of silicone oils, hydrocarbon oils, polar oils, such as higher fatty acids, ester oils, and natural animal and vegetable oils, semisynthetic oils, and/or fluorocarbon oils. Hydrocarbon oils and silicone oils are preferable. Because the oil-gelling agent contained in the cosmetic of the present invention has compatibility with even a silicone oil as the oil ingredient (B), the cosmetic is comfortable to use and can attain excellent usability and stability.

Examples of silicone oils include low-viscosity to high-viscosity linear or branched organopolysiloxanes, such as Dimethicone (INCI), Caprylyl Methicone (INCI), Phenyl Trimethicone (INCI), Hexyl Dimethicone (INCI), Hydrogen Dimethicone (INCI), and Diphenyl Dimethicone (INCI); cyclic organopolysiloxanes, such as Cyclotetrasiloxane (INCI), Cyclopentasiloxane (INCI), Cyclohexasiloxane (INCI), tetrahydrotetramethylcyclotetrasiloxane, and tetramethyltetraphenylcyclotetrasiloxane; branched organopolysiloxanes, such as tris(trimethylsiloxy)methylsilane and tetrakis(trimethylsiloxy)silane; silicone rubbers, such as amino-modified organopolysiloxanes, highly polymerized gum-like dimethylpolysiloxanes, gum-like amino-modified organopolysiloxanes, and gum-like dimethylsiloxane-methylphenylsiloxane copolymers; cyclic siloxane solutions of a silicone gum or rubber; Trimethylsiloxysilicate (INCI) and cyclic organopolysiloxane solutions of Trimethylsiloxysilicate; higher alkoxy-modified organopolysiloxanes, such as Stearoxy Dimethicone (INCI); higher fatty acid-modified organopolysiloxanes; alkyl-modified organopolysiloxanes, long-chain alkyl-modified organopolysiloxanes, fluorine-modified organopolysiloxanes, silicone resins, and dissolved silicone resins.

Hydrocarbon oils are exemplified by linear, branched, and also volatile hydrocarbon oils. Examples include Ozokerite (INCI), olefin oligomers, Isododecane (INCI), Hydrogenated Polyisobutene (INCI), Squalane (INCI), Squalene (INCI), Ceresin (INCI), Paraffin (INCI), Polyethylene (INCI), polyethylene-polypropylene wax, (ethylene/propylene/styrene) copolymer, (butylene/propylene/styrene) copolymer, Pristane (INCI), polyisobutylene, Microcrystalline Wax (INCI), and vaseline (INCI: Petrolatum).

Examples of higher fatty acids include Lauric Acid (INCI), Myristic Acid (INCI), Palmitic Acid (INCI), Stearic Acid (INCI), Behenic Acid (INCI), Undecylenic Acid (INCI), Oleic Acid (INCI), Linoleic Acid (INCI), Linolenic Acid (INCI), Arachidonic Acid (INCI), Eicosapentaenoic Acid (EPA) (INCI), Docosahexaenoic Acid (DHA) (INCI), Isostearic Acid (INCI), and Hydroxystearic Acid (INCI). Examples of higher alcohols include Lauryl Alcohol (INCI), Myristyl Alcohol (INCI), Cetanol (INCI), Stearyl Alcohol (INCI), Behenyl Alcohol (INCI), Oleyl Alcohol (INCI), Isostearyl Alcohol (INCI), Octyldodecanol (INCI), Cetearyl Alcohol (INCI), Decyltetradecanol (INCI), Cholesterol (INCI), Phytosterols (INCI), Batyl Alcohol (INCI), and oleyl glyceryl (INCI: Oleyl Glyceryl Ether).

Examples of ester oils include Diisobutyl Adipate (INCI), Diethylhexyl Adipate (INCI), diheptylundecyl adipate (INCI: Heptylundecyl Adipate), n-alkylene (20-30) monoisostearate glycols, Isocetyl Isostearate (INCI), Trimethylolpropane Triisostearate (INCI), ethylene glycol di-2-ethylhexanoate, cetyl 2-ethylhexanoate (INCI: Cetyl Ethylhexanoate), Trimethylolpropane Triethylhexanoate (INCI), Pentaerythrityl Tetraethylhexanoate (INCI), cetyl octanoate (INCI: Cetyl Ethylhexanoate), Octyldodecyl Myristate (INCI), Oleyl Oleate (INCI), Octyldodecyl Oleate (INCI), Decyl Oleate (INCI), neopentyl glycol dioctanoate (INCI: Neopentyl Glycol Diethylhexanoate), Neopentyl Glycol Dicaprate (INCI), Triethyl Citrate (INCI), Diethylhexyl Succinate (INCI), Amyl Acetate (INCI), Ethyl Acetate (INCI), Butyl Acetate (INCI), Isocetyl Isostearate (INCI), Butyl Stearate (INCI), Diisopropyl Sebacate (INCI), Diethylhexyl Sebacate (INCI), Cetyl Lactate (INCI), Myristyl Lactate (INCI), Isononyl Isononanoate (INCI), Isotridecyl Isononanoate (INCI), Isopropyl Palmitate (INCI), Ethylhexyl Palmitate (INCI), Hexyldecyl Palmitate (INCI), Cholesteryl Hydroxystearate (INCI), Isopropyl Myristate (INCI), Octyldodecyl Myristate (INCI), 2-hexyldecyl myristate, Myristyl Myristate (INCI), hexyldecyl dimethyloctanoate, ethyl laurate (INCI: Ethylhexyl Laurate), Hexyl Laurate (INCI), di(phytosterylloctyldodecyl) lauroyl glutamate (INCI: Phytosteryl/Octyldodecyl Lauroyl Glutamate), Isopropyl Lauroyl Sarcosinate (INCI), and Diisostearyl Malate (INCI). Examples of glyceride oils include Glyceryl Acetate (INCI), Triethylhexanoin (INCI), glyceryl triisostearate (INCI: Glyceryl Isostearate), Triisopalmitin (INCI), Glyceryl Stearate (INCI), Glyceryl Diisostearate (INCI), Trimyristin (INCI), and glyceryl (isostearate/myristate) (INCI: Isostearic/Myristic Glycerides).

Examples of natural animal and vegetable oils and semisynthetic oils include avocado oil (INCL: Persea Gratissima (Avocado) Oil), linseed oil (INCL: Linum Usitatissimum (Linseed) Seed Oil), almond oil (INCI: Prunus Amygdalus Dulcis (Sweet Almond) Oil), insect wax, perilla oil, olive oil, kapok wax, kaya nut oil, carnauba wax (INCI: Copernicia Cerifera (Carnauba) Wax), Shark Liver Oil (INCI), Cod Liver Oil (INCI), candelilla wax (INCI: Euphorbia Cerifera (Candelilla) Wax), beef tallow, neatsfoot oil, beef bone fat, hydrogenated beef tallow, Prunus Armeniaca (Apricot) Kernel Oil (INCI), spermaceti, Hydrogenated Palm Oil (INCI), wheatgerm oil (INCI: Triticum Vulgare (Wheat) Germ Oil), sesame oil (INCI: Sesamum Indicum (Sesame) Seed Oil), rice germ oil (INCI: Oryza Sativa (Rice) Germ Oil), rice bran oil (INCI: Oryza Sativa (Rice) Bran Oil), sugarcane wax (INCI: Saccharum Officinarum (Sugarcane) Wax), Camellia Kissi Seed Oil (INCI), safflower oil (INCI: Carthamus Tinctorius (Safflower) Seed Oil), shea butter (INCI: Butyrospermum Parkii (Shea Butter)), coconut oil (INCI: Cocos Nucifera (Coconut) Oil), cinnamon oil, jojoba wax (INCI: Simmondsia Chinensis (Jojoba) Seed Wax), Shellac Wax (INCI), Turtle Oil (INCI), soybean oil (INCI: Glycine Soja (Soybean) Oil), Camellia Sinensis Seed Oil (INCI), Camellia Japonica Seed Oil (INCI), evening primrose oil (INCI: Oenothera Biennis (Evening Primrose) Oil), corn oil (INCI: Zea Mays (Corn) Oil), Lard (INCI), rapeseed oil, tung oil, rice bran wax (INCI: Oryza Sativa (Rice) Bran Wax), Horse Fat (INCI), persic oil, palm oil (INCI: Elaeis Guineensis (Palm) Oil), palm kernel oil (INCI: Elaeis Guineensis (Palm) Kernel Oil), castor oil (INCI: Ricinus Communis (Castor) Seed Oil), Hydrogenated Castor Oil (INCI), castor oil fatty acid methyl ester, sunflower oil, grapeseed oil (INCI: Vitis Vinifera (Grape) Seed Oil), bayberry wax, Jojoba Oil (INCI), Hydrogenated Jojoba Oil (INCI), macadamia nut oil (INCI: Macadamia Integrifolia Seed Oil), Beeswax (INCI), Mink Oil (INCI), meadowfoam oil (INCI: Limnanthes Alba (Meadowfoam) Seed Oil), cottonseed oil (INCI: Gossypium (Cotton) Seed Oil), cotton wax, Japan wax (INCI: Rhus Succedanea Fruit Wax), Montan Wax (INCI), Hydrogenated Coconut Oil (INCI), glyceryl tricocoate, peanut oil (INCI: Arachis Hypogaea (Peanut) Oil), Lanolin (INCI), liquid lanolin (INCI: Lanolin Oil), Hydrogenated Lanolin (INCI), Lanolin Alcohol (INCI), Lanolin Wax (INCI), Acetylated Lanolin (INCI), Acetylated Lanolin Alcohol (INCI), Isopropyl Lanolate (INCI), POE lanolin alcohol ethers, POE lanolin alcohol acetates, polyethylene glycol lanolate, POE hydrogenated lanolin alcohol ethers, and egg yoke oil (INCI: Egg Oil). Here, "POE" stands for polyoxyethylene.

Fluorocarbon oils include perfluoropolyethers, Perfluorodecalin (INCI), and perfluorooctane.

When the oil ingredient (B) is added, the amount of the oil ingredient (B) contained in the cosmetic of the present invention is variable depending on the form of the cosmetic, but is preferably 1 to 98 wt%, and more preferably 1 to 50 wt% relative to the whole of the cosmetic.

### Ultraviolet-Absorbing Ingredients (C)

The cosmetic of the present invention may also include one, or two or more kinds of ultraviolet-absorbing ingredients. In this case, the cosmetic of the present invention can absorb ultraviolet rays as well as being comfortable to use and having excellent usability and staying power. The ultraviolet-absorbing ingredients include ultraviolet absorbers and ultraviolet-scattering agents. Examples of ultraviolet absorbers include benzoic acid ultraviolet absorbers, such as PABA (INCI), anthranilic acid ultraviolet absorbers, such as Methyl Anthranilate (INCI), salicylic acid ultraviolet absorbers, such as Methyl Salicylate (INCI), cinnamic acid ultraviolet absorbers, such as octyl methoxycinnamate (INCI: Ethylhexyl Methoxycinnamate), benzophenone ultraviolet absorbers, such as oxybenzone-1 (INCI: Benzophenone-1), urocanic acid ultraviolet absorbers, such as ethyl urocanate, and dibenzoylmethane ultraviolet absorbers, such as t-butyl methoxydibenzoylmethane (INCI: Butyl Methoxydibenzoylmethane). The earlier described silicone derivatives having functional groups with ultraviolet-absorbing properties may also be used. Ultraviolet absorbing and scattering agents are exemplified by powders that absorb and scatter ultraviolet light, including microparticulate titanium oxide, microparticulate iron-containing titanium oxide, microparticulate zinc oxide, microparticulate cerium oxide, and composites thereof. Among these, cinnamic acid ultraviolet absorbers, dibenzoylmethane ultraviolet absorbers, titanium oxide, and zinc oxide are preferable.

### Water (D)

Depending on the purpose of the cosmetic of the present invention, water may be added to the cosmetic. The addition of water in accordance with the purpose of use further enhances the usability of the cosmetic of the present invention. When water (D) is added, the amount thereof is preferably in the range of 95 wt% and less relative to the whole of the cosmetic.

### Surfactants (E)

The cosmetic of the present invention may further include one, or two or more kinds of surfactants. The addition of a surfactant in accordance with the purpose of use further enhances the usability of the cosmetic of the present invention. The surfactants added to the cosmetic of the present invention are not particularly limited and may be anionic, cationic, nonionic, or amphoteric surfactants. Any surfactants added to common cosmetics may be used.

Examples of anionic surfactants include fatty acid soaps, such as Sodium Stearate (INCI) and TEA-Palmitate (INCI); alkyl ether carboxylic acids and salts thereof; amino acid-fatty acid condensates; alkanesulfonates, alkenesulfonates, sulfonates of fatty acid esters, sulfonates of fatty acid amides, and formalin-condensed sulfonates; sulfate salts, such as alkyl sulfates, secondary higher alcohol sulfates, alkyl and aryl ether sulfates, sulfates of fatty acid esters, sulfates of fatty acid alkylolamides, and Sulfated Castor Oil (INCI); alkyl phosphates, ether phosphates, alkyl aryl ether phosphates, amide phosphates, N-acyl lactates, N-acyl sarcosinates, and N-acylamino acid surfactants. Examples of cationic surfactants include amine salts, such as alkylamine salts and polyamine and aminoalcohol fatty acid derivatives, alkyl quaternary ammonium salts, aromatic quaternary ammonium salts, pyridinium salts, and imidazolium salts.

Examples of nonionic surfactants include sorbitan fatty acid esters, glycerin fatty acid esters, polyglycerin fatty acid esters, propylene glycol fatty acid esters, polyethylene glycol fatty acid esters, sucrose fatty acid esters, methylglucoside fatty acid esters, alkyl polyglucosides, polyoxyethylene alkyl ethers, polyoxypropylene alkyl ethers, polyoxyethylene alkyl phenyl ethers, polyoxyethylene fatty acid esters, polyoxyethylene sorbitan fatty acid esters, polyoxyethylene sorbitol fatty acid esters, polyoxyethylene glycerin fatty acid esters, polyoxyethylene propylene glycol fatty acid esters, polyoxyethylene castor oil, polyoxyethylene hydrogenated castor oil, polyoxyethylene phytostanol ether, polyoxyethylene phytosterol ether, polyoxyethylene cholestanol ether, polyoxyethylene cholesterol ether, linear or branched polyoxyalkylene-modified organopolysiloxanes, linear or branched polyoxyalkylene/alkyl co-modified organopolysiloxanes, linear or branched polyglycerin-modified organopolysiloxanes, linear or branched polyglycerin/alkyl co-modified organopolysiloxanes, alkanolamides, sugar ethers, and sugar amides.

Examples of amphoteric surfactants include betaine, aminocarboxylic acid salts, imidazoline derivatives, and amidoamine compounds.

Of these surfactants, linear or branched organopolysiloxanes having polyoxyethylene chains on the molecule, linear or branched organopolysiloxanes having polyglycerin chains on the molecule, and surfactants which are alkyl co-modified organopolysiloxanes of each are preferable. Examples of commercial products include, without particular limitation, KF-6011, KF-6011P, KF-6043, KF-6012, KF-6013, KF-6015, KF-6016, KF-6017, KF-6028, KF-6028P, KF-6038, KF-6100, KF-6104, KF-6105, and KF-6106 (all from Shin-Etsu Chemical Co., Ltd.). Surfactants having a hydrophilic-lipophilic balance (HLB) of 2 to 10 are preferable. When the surfactant (E) is added, the amount thereof is preferably 0.1 to 20 wt%, and more preferably 0.2 to 10 wt%, relative to the whole of the cosmetic.

### Powders (F)

The cosmetic of the present invention may further include one, or two or more kinds of powders. Any powders that are used in common cosmetics may be used regardless of shapes (spherical, needle-like, platy, etc.), particle sizes (aerosols, microparticles, pigment grades, etc.), and particle structures (porous, nonporous, etc.). Exemplary powders include inorganic powders, organic powders, surfactant metal salt powders, and colorants, such as color pigments, pearlescent pigments, coal tar dyes, metal powder pigments, natural colors, and dyes.

Examples of inorganic powders include titanium oxide (INCI: Titanium Dioxide), zirconium oxide (INCI: Zirconium Dioxide), Zinc Oxide (INCI), Cerium Oxide (INCI), magnesium oxide, barium sulfate, calcium sulfate, magnesium sulfate, calcium carbonate, magnesium carbonate, Talc (INCI), Mica (INCI), Kaolin (INCI), sericite, synthetic mica, phlogopite, red lepidolite, biotite, lepidolite, Silica (INCI), Aluminum Silicate (INCI), Magnesium Silicate (INCI), Magnesium Aluminum Silicate (INCI), Calcium Silicate (INCI), barium silicate, strontium silicate, metal salts of tungstic acid, Hydroxyapatite (INCI), vermiculite, Hygilite, Bentonite (INCI), Montmorillonite (INCI), Hectorite (INCI), Zeolite (INCI), dibasic calcium phosphate, Alumina (INCI), Aluminum Hydroxide (INCI), Boron Nitride (INCI), and Silica Silylate (INCI).

Examples of organic powders include polyamide powder, polyacrylic acid/polyacrylate ester powder, polyester powder, polyethylene powder, polypropylene powder, polystyrene powder, polyurethane, benzoguanamine powder, polymethyl benzoguanamine powder, tetrafluoroethylene powder, polymethyl methacrylate powder, Cellulose (INCI), silk powder, nylon powder, Nylon-12 (INCI), Nylon-6 (INCI), crosslinked spherical dimethylpolysiloxane fine powders having a structure in which the dimethylpolysiloxane is crosslinked, crosslinked spherical polymethylsilsesquioxane fine powder, a fine powder in which crosslinked spherical organopolysiloxane rubber surfaces are coated with polymethylsilsesquioxane particles, hydrophobized silica, styrene-acrylic acid copolymers, divinylbenzene-styrene copolymers, vinyl resins, urea resins, phenolic resins, fluorocarbon resins, silicone resins, acrylic resins, melamine resins, epoxy resins, polycarbonate resins, microcrystalline fiber powder, starch powder, fatty acid starch derivative powder, and Lauroyl Lysine (INCI).

Examples of surfactant metal salt powders (metal soaps) include Zinc Undecylenate (INCI), Aluminum Isostearate (INCI), Zinc Stearate (INCI), Aluminum Stearate (INCI), Calcium Stearate (INCI), Magnesium Stearate (INCI), Zinc Myristate (INCI), Magnesium Myristate (INCI), Sodium Zinc Cetyl Phosphate (INCI), calcium cetyl phosphate, Zinc Palmitate (INCI), aluminum palmitate, and Zinc Laurate (INCI).

Specific examples of color pigments include inorganic red pigments, such as iron oxide, iron hydroxide, and iron titanate; inorganic brown pigments, such as γ-iron oxide; inorganic yellow pigments, such as yellow iron oxide and yellow ocher; inorganic black pigments, such as black iron oxide and Carbon Black (INCI); inorganic violet pigments, such as Manganese Violet (INCI) and cobalt violet; inorganic green pigments, such as chromium hydroxide (INCI: Chromium Hydroxide Green), chromium oxide (INCI: Chromium Oxide Greens), cobalt oxide, and cobalt titanate (INCI: Cobalt Titanium Oxide); inorganic blue pigments, such as Prussian blue and ultramarine blue; lakes of coal tar dyes, lakes of natural dyes, and synthetic resin powders that are composites of these powders.

Specific examples of pearlescent pigments include titanium oxide-coated mica, bismuth oxychloride, titanium oxide-coated bismuth oxychloride, titanium oxide-coated talc, natural pearl essence, and titanium oxide-coated colored mica. Examples of metal powder pigments include aluminum powder, copper powder, and stainless steel powder.

Examples of coal tar dyes include red No. 3, red No. 104, red No. 106, red No. 201, red No. 202, red No. 204, red No. 205, red No. 220, red No. 226, red No. 227, red No. 228, red No. 230, red No. 401, red No. 505, yellow No. 4, yellow No. 5, yellow No. 202, yellow No. 203, yellow No. 204, yellow No. 401, blue No. 1, blue No. 2, blue No. 201, blue No. 404, green No. 3, green No. 201, green No. 204, green No. 205, orange No. 201, orange No. 203, orange No. 204, orange No. 206, and orange No. 207. Examples of natural colors include powders selected from carminic acid, Laccaic Acid (INCI), carthamin, brazilin, and crocin.

Of these powders, crosslinked spherical dimethylpolysiloxane fine powders having a structure crosslinked with dimethylpolysiloxane, crosslinked spherical polymethylsilsesquioxane fine powder, a fine powder in which crosslinked spherical polysiloxane rubber surfaces are coated with polymethylsilsesquioxane particles, a fine powder in which crosslinked spherical diphenyl polysiloxane rubber surfaces are coated with polymethylsilsesquioxane particles, and hydrophobized silica are preferable in the present invention. Powders and colorants having fluorine groups may also be used. Commercial products include KMP-590, KSP-100, KSP-101, KSP-102, KSP-105, and KSP-300 (all from Shin-Etsu Chemical Co., Ltd.).

As long as the advantageous effects of the present invention are not impaired, the powders may be formed into composites or may be treated with, for example, general oils, silicone oils, fluorine compounds, or surfactants. The powders may or may not be surface-treated beforehand by way of, for example, fluorine compound treatment, silicone resin treatment, pendant treatment, silane-coupling agent treatment, titanium-coupling agent treatment, oil treatment, N-acylated lysine treatment, polyacrylic acid treatment, metal soap treatment, amino acid treatment, inorganic compound treatment, plasma treatment, or mechanochemical treatment. One, or two or more such treatments may be used as required. When the powder (F) is added, the amount thereof is preferably 99 wt% or less relative to the whole of the cosmetic. When, in particular, the cosmetic is a powdery cosmetic, the amount is preferably 80 to 99 wt% of the whole of the cosmetic.

### Compounds (G) having an alcoholic hydroxyl group in the molecular structure

The cosmetic of the present invention may further include one, or two or more kinds of compounds having an alcoholic hydroxyl group in the molecular structure. C₁₂ or higher alcohol compounds for constituting the monomer units in the copolymer are excluded from the compounds having an alcoholic hydroxyl group. Examples of such compounds include lower alcohols, such as ethanol (INCI: Alcohol) and Isopropyl Alcohol (INCI); sugar alcohols, such as Sorbitol (INCI) and Maltose (INCI); sterols, such as Cholesterol (INCI), Beta-Sitosterol (INCI), Phytosterols (INCI), and Lanosterol (INCI); and polyhydric alcohols, such as BG (INCI: Butylene Glycol), PG (INCI: Propylene Glycol), DPG (INCI: Dipropylene Glycol), and Pentylene Glycol (INCI). In usual cases, water-soluble monohydric alcohols and water-soluble polyhydric alcohols are frequently used. When the compound (G) having an alcoholic hydroxyl group in the molecular structure is added, the amount thereof is preferably 98 wt% or less of the whole of the cosmetic.

### Water-soluble or water-swellable polymeric compounds (H)

The cosmetic of the present invention may further include one, or two or more kinds of water-soluble or water-swellable polymeric compounds. Examples of these water-soluble or water-swellable polymeric compounds include plant-derived polymeric compounds, such as gum arabic (INCI: Acacia Senegal Gum), tragacanth gum tree gum (INCI: Astragalus Gummifer Gum), galactan, locust bean gum (INCI: Ceratonia Siliqua Gum), guar gum (INCI: Cyamopsis Tetragonoloba (Guar) Gum), karaya gum (INCI: Sterculia Urens Gum), carrageenan (INCI: Chondrus Crispus (Carrageenan)), Pectin (INCI), Agar (INCI), quince seed (INCI: Pyrus Cydonia Seed), rice starch (INCI: Oryza Sativa (Rice) Starch), wheat starch (INCI: Triticum Vulgare (Wheat) Starch), potato starch (INCI: Solanum Tuberosum (Potato) Starch), and tragacanth (INCI: Astragalus Gummifer Gum); microorganism-derived polymeric compounds, such as Xanthan Gum (INCI), Dextran (INCI), Succinoglycan (INCI), and Pullulan (INCI); animal-derived polymeric compounds, such as Collagen (INCI), Casein (INCI), albumin, and Gelatin (INCI); starch-based polymeric compounds, such as Sodium Carboxymethyl Starch (INCI) and Hydroxypropyl Starch (INCI); cellulosic polymeric compounds, such as Methylcellulose (INCI), Ethylcellulose (INCI), Hydroxypropyl Methylcellulose (INCI), carboxymethylcellulose, hydroxymethylcellulose, Hydroxypropylcellulose (INCI), Nitrocellulose (INCI), Sodium Cellulose Sulfate (INCI), sodium carboxymethylcellulose, Microcrystalline Cellulose (INCI), and cellulose powder; alginic acid-based polymeric compounds, such as sodium alginate (INCI: Algin), PG alginate (INCI: Propylene Glycol Alginate); vinyl-based polymeric compounds, such as polyvinyl methyl ether and carboxyvinyl polymer; polyoxyethylene-based polymeric compounds; polyoxyethylene-polyoxypropylene copolymer-based polymeric compounds; acrylic polymers, such as Sodium Polyacrylate (INCI), Polyethylacrylate (INCI), Polyacrylamide (INCI), and Acrylamide/Sodium Acryloyldimethyltaurate Copolymer (INCI); other synthetic water-soluble polymeric compounds, such as polyethyleneimine and cationic polymers; and inorganic water-soluble polymeric compounds, such as Bentonite (INCI), Magnesium Aluminum Silicate (INCI), Montmorillonite (INCI), beidellite, nontronite, saponite, Hectorite (INCI), and Silica (INCI). Film-forming agents, such as Polyvinyl Alcohol (INCI) and PVP (INCI), are also included among these water-soluble polymeric compounds. The amount in which the water-soluble or water-swellable polymeric compound is added is preferably 25 wt% or less of the whole of the cosmetic.

### Compositions (I) of a hydrophilic group-free crosslinked organopolysiloxane polymer and a liquid oil

The cosmetic of the present invention may further include one, or two or more kinds of compositions (I) of a hydrophilic group-free crosslinked organopolysiloxane polymer and a liquid oil. The crosslinked organopolysiloxane polymer may be obtained by reacting an alkyl hydrogen polysiloxane with a hydrosilylatable unsaturated group-containing crosslinking agent.

Examples of the alkyl hydrogen polysiloxane include methyl hydrogen polysiloxanes having linear or partially branched units, and methyl hydrogen polysiloxanes to which have been grafted alkyl chains of from 6 to 20 carbon atoms. The molecule must have an average of two or more silicon-bonded hydrogen atoms thereon.

Examples of the crosslinking agent include compounds having on the molecule two or more hydrosilylatable carbon-carbon double bonds, such as methyl vinyl polysiloxane and α,ω-alkenyldienes.

The crosslinked organopolysiloxane polymer thus obtained is swollen with, for example, its own weight or more of a liquid oil, for example, a low-viscosity silicone having a kinematic viscosity of 0.65 mm²/s (25°C) to 100.0 mm²/s (25°C), a hydrocarbon oil, such as liquid paraffin, squalane, or isododecane, a glyceride oil, such as trioctanoin, or an ester oil.

In the present invention, the "kinematic viscosity" is a value measured at 25°C using a Cannon-Fenske viscometer described in JIS Z8803: 2011.

Compositions of these crosslinked organopolysiloxane polymers and liquid oils are available as commercial products. Examples include KSG-15, KSG-16, KSG-18, KSG-1610, and USG-103 that are pastes with a silicone oil; and USG-106, KSG-41, KSG-42, KSG-43, KSG-44, and KSG-810 that are pastes with a hydrocarbon oil or a triglyceride oil. These are all products of Shin-Etsu Chemical Co., Ltd. When the composition (I) of a hydrophilic group-free crosslinked organopolysiloxane polymer and a liquid oil is added, the amount thereof is preferably 0.1 to 50 wt%, and more preferably 1 to 30 wt% of the whole of the cosmetic.

### Compositions (J) of a hydrophilic group-containing crosslinked organopolysiloxane polymer and a liquid oil

The cosmetic of the present invention may further include one, or two or more kinds of compositions of a hydrophilic group-containing crosslinked organopolysiloxane polymer and a liquid oil. The hydrophilic group is preferably a polyether group or a polyglyceryl group. A polyether group and/or polyglycerol group-containing crosslinked organopolysiloxane polymer may be obtained by reacting an alkyl hydrogen polysiloxane with a hydrosilylatable unsaturated group-containing crosslinking agent. Examples of the alkyl hydrogen polysiloxane include methyl hydrogen polysiloxanes to which have been grafted polyoxyethylene chains and methyl hydrogen polysiloxanes to which have been grafted polyglycerol chains. The molecule must have an average of two or more silicon-bonded hydrogen atoms thereon.

Examples of the crosslinking agent include methylvinylpolysiloxane, and compounds having on the molecule two or more hydrosilylatable carbon-carbon double bonds, such as α,ω-alkenyldienes, glycerol triallyl ether, polyoxyalkynylated glycerol triallyl ether, trimethylolpropane triallyl ether, and polyoxyalkynylated trimethylolpropane trially ether. The crosslinked product obtained by reacting these is a compound having at least one hydrophilic group.

The resulting crosslinked organopolysiloxane polymer is swollen with, for example, its own weight or more of a liquid oil, for example, a low-viscosity silicone having a kinematic viscosity of 0.65 mm²/s (25°C) to 100.0 mm²/s (25°C), a hydrocarbon oil, such as liquid paraffin, squalane, or isododecane, a glyceride oil, such as trioctanoin, or an ester oil.

These crosslinked organopolysiloxanes are available as commercial products. Examples include KSG-210, KSG-240, and KSG-710 that are pastes with a silicone oil; and KSG-310, KSG-320, KSG-330, KSG-340, KSG-820, KSG-830, and KSG-840 that are pastes with a hydrocarbon oil or a triglyceride oil. These are all products of Shin-Etsu Chemical Co., Ltd. When the composition (J) of a hydrophilic group-containing crosslinked organopolysiloxane polymer and a liquid oil is added, the amount thereof is preferably 0.1 to 50 wt%, and more preferably 1 to 30 wt% of the whole of the cosmetic.

### Silicone resins (K)

The cosmetic of the present invention may further include one, or two or more kinds of silicone resins (K). The silicone resin is preferably selected from the group consisting of silicone network compounds containing SiO_{4/2} and/or R'SiO_{3/2} (wherein R' is an alkyl group) units, linear acrylic/silicone grafts, and block copolymers thereof. The linear acrylic/silicone graft or the block copolymer may have one or more kinds of moieties selected from pyrrolidone moieties, long-chain alkyl moieties, polyoxyalkylene moieties, fluoroalkyl moieties, and anionic moieties of carboxylic acids and the like. Examples of commercial products include, without particular limitation, the following dissolved in a silicone oil, a hydrocarbon oil, or an alcohol: KP-541, KP-543, KP-545, KP-549, KP-550, KP-571, KP-575, and KP-581 (all from Shin-Etsu Chemical Co., Ltd.).

The silicone network compound is preferably a silicone network compound denoted as MQ, MDQ, MT, MDT, or MDTQ. Here, M, D, T, and Q respectively stand for R³SiO_{1/2} units, R²SiO_{2/2} units, RSiO_{3/2} units, and SiO_{4/2} units. The silicone network compound may include on the molecule one or more kinds of moieties selected from pyrrolidone moieties, long-chain alkyl moieties, polyoxyalkylene moieties, fluoroalkyl moieties, and amino moieties. Examples of commercial products include, without particular limitation, KF-7312J, KF-7312K, and KF-7312T (all from Shin-Etsu Chemical Co., Ltd.).

The silicone resin (K) may be used as a solution in a low-viscosity silicone oil, a volatile silicone oil, or other solvent. When the resin is added in any of the manners described above, the amount of the resin is preferably 0.1 to 20 wt%, and more preferably 1 to 10 wt% of the whole of the cosmetic.

### Silicone waxes (L)

The cosmetic of the present invention may also include a silicone wax (L) depending on the purpose thereof. This silicone wax is exemplified by polylactone-modified polysiloxanes in which a polylactone that is a ring-opening polymer of a five-membered or larger lactone compound is bonded, and acrylic-modified polysiloxanes containing on the molecule one or more kinds of functional groups selected from pyrrolidone groups, long-chain alkyl groups, polyoxyalkylene groups, fluoroalkyl groups, and anionic groups of carboxylic acids and the like. Examples of commercial products of waxes containing a long-chain alkyl group include KP-561P and KP-562P (both from Shin-Etsu Chemical Co., Ltd.).

Examples of silicone waxes include silicone-modified olefin waxes obtained by addition reacting an olefin wax with an organohydrogen polysiloxane having one or more SiH bonds on the molecule. The olefin wax is one obtained by copolymerizing ethylene and one or more kinds of dienes, or one obtained by copolymerizing ethylene, one or more kinds of olefins selected from C₃₋₁₂ α-olefins, and one or more kinds of dienes. The diene is preferably vinyl norbornene.

When the silicone wax is used, the amount thereof is preferably 0.1 to 20 wt%, and more preferably 1 to 10 wt% of the whole of the cosmetic.

### Other ingredients

The cosmetic of the present invention may further include ingredients used in common cosmetics without impairing the advantageous effects of the present invention. Examples of such ingredients include oil-soluble gelling agents, antiperspirants, moisturizers, antimicrobial preservatives, salts, antioxidants, skin beautifying ingredients (whitening agents, cell activators, skin roughness improvers, circulation promoting ingredients, skin astringents, antiseborrheic agents, etc.), vitamins, amino acids, and hair-fixing polymeric compounds.

For example, the oil-soluble gelling agent may be one, or two or more kinds of oil-soluble gelling agents selected from metal soaps, such as Aluminum Stearate (INCI), Magnesium Stearate (INCI), and Zinc Myristate (INCI); amino acid derivatives, such as Lauroyl Glutamic Acid (INCI); dextrin fatty acid esters, such as Dextrin Palmitate (INCI), Dextrin Isostearate (INCI), and Dextrin Palmitate/Ethylhexanoate (INCI); sucrose fatty acid esters, such as Sucrose Palmitate (INCI) and Sucrose Stearate (INCI); fructooligosaccharide fatty acid esters, such as Fructooligosaccharide Caprylate (INCI) and Fructooligosaccharide Hexyldecanoate (INCI); benzylidene derivatives of sorbitol, such as monobenzylidene sorbitol and dibenzylidene sorbitol; and organic-modified clay minerals, such as dimethylbenzyl dodecylammonium montmorillonite clay and dimethyldioctadecylammonium montmorillonite clay.

For example, the antiperspirant may be one, or two or more kinds of antiperspirants selected from Aluminum Chlorohydrate (INCI), Aluminum Chloride (INCI), aluminum sesquichlorohydrate, zirconium hydroxychloride, aluminum-zirconium hydroxychloride, and aluminum-zirconium glycine complexes.

For example, the moisturizer may be one, or two or more kinds of moisturizers selected from Glycerin (INCI), Sorbitol (INCI), PG (INCI), DPG (INCI), BG (INCI), Pentylene Glycol (INCI), Glucose (INCI), Xylitol (INCI), Maltitol (INCI), polyethylene glycol, Hyaluronic Acid (INCI), chondroitin sulfate, pyrrolidonecarboxylic acid salts, polyoxyethylene methyl glucoside, and polyoxypropylene methyl glucoside.

For example, the antimicrobial preservative may be one, or two or more kinds of antimicrobial preservatives selected from alkyl p-hydroxybenzoates, Benzoic Acid (INCI), Sodium Benzoate (INCI), Sorbic Acid (INCI), Potassium Sorbate (INCI), and Phenoxyethanol (INCI). Examples of antimicrobial agents include benzoic acid, salicylic acid, Phenol (INCI), sorbic acid, alkyl p-hydroxybenzoates, p-Chloro-m-Cresol (INCI), Hexachlorophene (INCI), Benzalkonium Chloride (INCI), Chlorhexidine Dihydrochloride (INCI), Triclocarban (INCI), photosensitizers, and phenoxyethanol.

Exemplary salts include inorganic salts, organic acid salts, amine salts, and amino acid salts. One, or two or more kinds of salts may be selected from inorganic salts, such as the sodium, potassium, magnesium, calcium, aluminum, zirconium, and zinc salts of inorganic acids, such as hydrochloric acid, sulfuric acid, carbonic acid, and nitric acid; salts of organic acids, such as Acetic Acid (INCI), Dehydroacetic Acid (INCI), Citric Acid (INCI), Malic Acid (INCI), Succinic Acid (INCI), Ascorbic Acid (INCI), and Stearic Acid (INCI); and salts of amines, such as TEA (INCI: Triethanolamine) and salts of amino acids, such as Glutamic Acid (INCI). In addition, use can be made of salts of Hyaluronic Acid (INCI), chondroitin sulfuric acid, and the like; Aluminum Zirconium Tetrachlorohydrate (INCI); and also acid-alkali neutralization salts used in cosmetic formulations.

Examples of antioxidants include Tocopherol (INCI), BHA (INCI), BHT (INCI), and Phytic Acid (INCI). Examples of pH adjustors include Lactic Acid (INCI), Citric Acid (INCI), Glycolic Acid (INCI), Succinic Acid (INCI), Tartaric Acid (INCI), Malic Acid (INCI), Potassium Carbonate (INCI), Sodium Bicarbonate (INCI), and Ammonium Bicarbonate (INCI). Examples of chelating agents include Alanine (INCI), Disodium EDTA (INCI), Sodium Polyphosphate (INCI), Sodium Metaphosphate (INCI), and Phosphoric Acid (INCI). Examples of algefacients include Menthol (INCI) and camphor. Examples of anti-inflammatory agents include Allantoin (INCI), Glycyrrhizic Acid (INCI) and salts thereof, Glycyrrhetinic Acid (INCI), Stearyl Glycyrrhetinate (INCI), tranexamic acid, and Azulene (INCI). One, or two or more kinds of antioxidants selected from the above may be used.

For example, the skin beautifying ingredient may be one, or two or more kinds of skin beautifying ingredients selected from whitening agents, such as Placental Extract (INCI), Albutin (INCI), Glutathione (INCI), and Saxifraga Sarmentosa Extract (INCI); cell activators, such as Royal Jelly (INCI), photosensitizers, cholesterol derivatives, and calf blood extract; skin roughness improvers; circulation promoting ingredients, such as nonanoic acid vanillylamide, Benzyl Nicotinate (INCI), β-butoxyethyl nicotinate, Capsaicin (INCI), Acetyl Zingerone (INCI), cantharides tincture, Ichthammol (INCI), Caffeine (INCI), tannic acid, Borneol (INCI), Tocopheryl Nicotinate (INCI), inositol hexanicotinate, cyclandelate, cinnarizine, tolazoline, acetylcholine, verapamil, cepharanthine, and Oryzanol (INCI); skin astringents, such as Zinc Oxide (INCI) and Tannic Acid (INCI); and antiseborrheic agents, such as Sulfur (INCI) and thianthrol.

For example, the vitamin may be one, or two or more kinds of vitamins selected from vitamin A oil and vitamin A compounds, such as Retinol (INCI), Retinyl Acetate (INCI), and Retinyl Palmitate (INCI); vitamin B compounds, including vitamin B₂ compounds, such as Riboflavin (INCI), Riboflavin Tetrabutyrate (INCI), and Disodium Flavine Adenine Dinucleotide (INCI), vitamin B₆ compounds, such as Pyrodoxine HCl (INCI), pyridoxine dioctanoate, and Pyridoxine Dipalmitate (INCI), vitamin B12 and derivatives thereof, and vitamin B15 and derivatives thereof; vitamin C compounds, such as Ascorbic Acid (INCI), Ascorbyl Dipalmitate (INCI), Disodium Ascorbyl Sulfate (INCI), and dipotassium L-ascorbyl diphosphate; vitamin D compounds, such as Ergocalciferol (INCI) and Cholecalciferol (INCI); vitamin E compounds, such as Tocopherol (INCI), Tocopheryl Acetate (INCI), Tocopheryl Nicotinate (INCI), and Tocopheryl Succinate (INCI); vitamin P compounds, such as Biotin (INCI) and Rutin (INCI); nicotinic acids, such as Niacin (INCI), Benzyl Nicotinate (INCI), and Niacinamide (INCI); and pantothenic acids, such as Calcium Pantothenate (INCI), Panthenol (INCI), pantothenyl ethyl ether, and acetyl pantothenyl ethyl ether.

Examples of amino acids include Glycine (INCI), Valine (INCI), Leucine (INCI), Isoleucine (INCI), Serine (INCI), Threonine (INCI), Phenylalanine (INCI), Arginine (INCI), Lysine (INCI), Aspartic Acid (INCI), Glutamic Acid (INCI), Cystine (INCI), Cysteine (INCI), Methionine (INCI), and Tryptophan (INCI). Examples of nucleic acids include DNA (INCI). Examples of hormones include Estradiol (INCI) and Ethynyl Estradiol (INCI). One, or two or more kinds of amino acids selected from the above may be used.

Examples of hair-fixing polymeric compounds include various amphoteric, anionic, cationic, and nonionic polymeric compounds. One, or two or more kinds of hair-fixing polymeric compounds may be selected from polyvinylpyrrolidone-based polymeric compounds, such as PVP (INCI) and (VP/VA) Copolymer (INCI); acidic vinyl ether-based polymeric compounds, such as (methyl vinyl ether/maleic acid) copolymer (INCI: PVM/MA Copolymer); acidic vinyl polyacetate polymeric compounds, such as VA/Crotonates Copolymer (INCI); acidic acrylic polymeric compounds, such as (meth)acrylic acid/alkyl (meth)acrylate copolymers and (meth)acrylic acid/alkyl (meth)acrylate/alkyl acrylamide copolymers; and amphoteric acrylic polymeric compounds, such as N-methacryloylethyl-N,N-dimethylammonium/α-N-methylcarboxybetaine/alkyl (meth)acrylate copolymers and (octyl acrylamide/hydroxypropyl acrylate/butylaminoethyl methacrylate) copolymer. Naturally occurring polymeric compounds, such as cellulose and derivatives thereof, keratin and collagen, and derivatives thereof may also be suitably used. In addition to the above, resins, fragrances, pH adjustors, chelating agents, algefacients, anti-inflammatory agents, nucleic acids, hormones, and inclusion compounds may be included as other ingredients.

The cosmetic in the present invention is exemplified by skin care cosmetics, such as milky lotions, creams, cleansing products, packs, massage products, beauty essences, beauty oils, cleansing agents, deodorants, hand creams, lip creams, and wrinkle concealers; makeup cosmetics, such as makeup bases, concealers, face powders, liquid foundations, oil-based foundations, rouges, eye shadows, mascaras, eye liners, eyebrows, and lipsticks; hair cosmetics, such as shampoos, rinses, treatments, and setting agents; and ultraviolet-protecting cosmetics, such as antiperspirants, sunscreen oils, sunscreen lotions, and sunscreen creams. These cosmetics are formed by blending the cosmetic ingredients described hereinabove.

The shape of the cosmetic may be selected from among various shapes, such as liquids, emulsions, creams, solids, pastes, gels, powders, compacts, multilayers, mousses, sprays, and sticks.

The form of the cosmetic may be selected from among various forms, such as aqueous, oil-based, water-in-oil emulsions, oil-in-water emulsions, nonaqueous emulsions, and multi-emulsions, such as W/O/W and O/W/O.

### EXAMPLES

The present invention is illustrated more fully below by way of Examples and Comparative Examples, although the present invention is not limited by these Examples. In the following Examples, unless noted otherwise, references to "%" in the composition signify percent by weight and references to "ratio" signify a weight ratio.

### [Synthesis Example 1] Synthesis of amphiphilic methacrylic monomer (AM1)

Palmitic acid (53.2 g), tetrabutylammonium bromide (6.71 g), and toluene (60 mL) were added to a 300 mL separable flask, and the mixture was stirred at 90°C. Glycidyl methacrylate (29.6 g) was added thereto dropwise through a dropping funnel. Subsequently, the mixture was stirred at 90°C for 4 hours. After the reaction, the reaction mixture was washed with a saturated aqueous NaCl solution three times, and the organic phase was recovered and was dehydrated with sodium sulfate. The sodium sulfate was filtered, and the solvent was distilled off. Thus, an amphiphilic methacrylic monomer (AM1) represented by general formula (4) below was obtained.

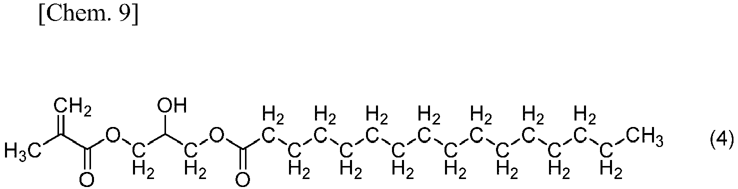

### [Synthesis Example 2] Synthesis of amphiphilic methacrylic monomer (AM2)

Stearic acid (100.1 g), tetrabutylammonium bromide (11.4 g), and toluene (100 mL) were added to a 300 mL separable flask, and the mixture was stirred at 90°C. Glycidyl methacrylate (50.0 g) was added thereto dropwise through a dropping funnel. Subsequently, the mixture was stirred at 90°C for 4 hours. After the reaction, the reaction mixture was washed with a saturated aqueous NaCl solution three times, and the organic phase was recovered and was dehydrated with sodium sulfate. The sodium sulfate was filtered, and the solvent was distilled off. Thus, an amphiphilic methacrylic monomer (AM2) represented by general formula (5) below was obtained.

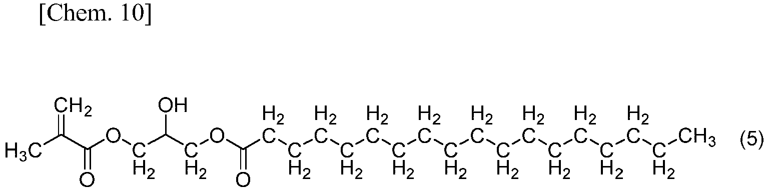

### [Example 1]

Isopropyl alcohol (23.3 g) was added to a separable flask and was heated at 100°C. AM1 (31.6 g), 2-hydroxyethyl methacrylate (1.7 g), isopropyl alcohol (16.6 g), and PERBUTYL O (manufactured by NOF Corporation) (2.0 g) were mixed together, and the mixture was added dropwise to the separable flask through a dropping funnel over a period of 1 hour. After the dropwise addition, the flask contents were stirred for 2 hours, and PERBUTYL O (0.3 g) was further added. The system was further stirred for 3 hours, bringing the reaction to completion. Subsequently, the solvent was driven off under reduced pressure (110°C, ≤ 1 kPa), giving the target copolymer. The number average molecular weight Mn was 29,595.

### [Example 2]

Isopropyl alcohol (23.3 g) was added to a separable flask and was heated at 100°C. AM2 (31.6 g), 2-hydroxyethyl methacrylate (1.7 g), isopropyl alcohol (16.6 g), and PERBUTYL O (manufactured by NOF Corporation) (2.0 g) were mixed together, and the mixture was added dropwise to the separable flask through a dropping funnel over a period of 1 hour. After the dropwise addition, the flask contents were stirred for 2 hours, and PERBUTYL O (0.3 g) was further added. The system was further stirred for 3 hours, bringing the reaction to completion. Subsequently, the solvent was driven off under reduced pressure (110°C, ≤ 1 kPa), giving the target copolymer. The number average molecular weight Mn was 29,578.

### [Example 3]

Isopropyl alcohol (23.3 g) was added to a separable flask and was heated at 100°C. AM2 (30.0 g), 2-hydroxyethyl methacrylate (3.3 g), isopropyl alcohol (16.6 g), and PERBUTYL O (manufactured by NOF Corporation) (2.0 g) were mixed together, and the mixture was added dropwise to the separable flask through a dropping funnel over a period of 1 hour. After the dropwise addition, the flask contents were stirred for 2 hours, and PERBUTYL O (0.3 g) was further added. The system was further stirred for 3 hours, bringing the reaction to completion. Subsequently, the solvent was driven off under reduced pressure (110°C, ≤ 1 kPa), giving the target copolymer. The number average molecular weight Mn was 34,493.

### [Example 4]

Isopropyl alcohol (23.3 g) was added to a separable flask and was heated at 100°C. AM2 (28.3 g), 2-hydroxyethyl methacrylate (5.0 g), isopropyl alcohol (16.6 g), and PERBUTYL O (manufactured by NOF Corporation) (2.0 g) were mixed together, and the mixture was added dropwise to the separable flask through a dropping funnel over a period of 1 hour. After the dropwise addition, the flask contents were stirred for 2 hours, and PERBUTYL O (0.3 g) was further added. The system was further stirred for 3 hours, bringing the reaction to completion. Subsequently, the solvent was driven off under reduced pressure (110°C, ≤ 1 kPa), giving the target copolymer. The number average molecular weight Mn was 37,010.

### [Example 5]

Isopropyl alcohol (23.3 g) was added to a separable flask and was heated at 100°C. AM1 (19.0 g), 2-hydroxyethyl methacrylate (1.0 g), silicone macromer (13.3 g) represented by general formula (6) below, isopropyl alcohol (16.6 g), and PERBUTYL O (manufactured by NOF Corporation) (2.0 g) were mixed together, and the mixture was added dropwise to the separable flask through a dropping funnel over a period of 1 hour. After the dropwise addition, the flask contents were stirred for 2 hours, and PERBUTYL O (0.3 g) was further added. The system was further stirred for 3 hours, bringing the reaction to completion. Subsequently, the solvent was driven off under reduced pressure (110°C, ≤ 1 kPa), giving the target copolymer. The number average molecular weight Mn was 35,450.

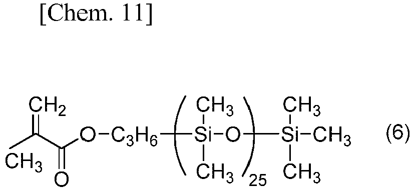

### [Comparative Example 1]

Isopropyl alcohol (23.3 g) was added to a separable flask and was heated at 100°C. AM1 (26.6 g), 2-hydroxyethyl methacrylate (6.7 g), isopropyl alcohol (16.6 g), and PERBUTYL O (manufactured by NOF Corporation) (2.0 g) were mixed together, and the mixture was added dropwise to the separable flask through a dropping funnel over a period of 1 hour. After the dropwise addition, the flask contents were stirred for 2 hours, and PERBUTYL O (0.3 g) was further added. The system was further stirred for 3 hours, bringing the reaction to completion. Subsequently, the solvent was driven off under reduced pressure (110°C, ≤ 1 kPa), giving the target copolymer. The number average molecular weight Mn was 30,128.

### [Comparative Example 2]

Isopropyl alcohol (23.3 g) was added to a separable flask and was heated at 100°C. AM2 (26.6 g), 2-hydroxyethyl methacrylate (6.7 g), isopropyl alcohol (16.6 g), and PERBUTYL O (manufactured by NOF Corporation) (2.0 g) were mixed together, and the mixture was added dropwise to the separable flask through a dropping funnel over a period of 1 hour. After the dropwise addition, the flask contents were stirred for 2 hours, and PERBUTYL O (0.3 g) was further added. The system was further stirred for 3 hours, bringing the reaction to completion. Subsequently, the solvent was driven off under reduced pressure (110°C, ≤ 1 kPa), giving the target copolymer. The number average molecular weight Mn was 35,280.

### [Comparative Example 3]

Isopropyl alcohol (23.3 g) was added to a separable flask and was heated at 100°C. Stearyl methacrylate (30.0 g), 2-hydroxyethyl methacrylate (3.3 g), isopropyl alcohol (16.6 g), and PERBUTYL O (manufactured by NOF Corporation) (2.0 g) were mixed together, and the mixture was added dropwise to the separable flask through a dropping funnel over a period of 1 hour. After the dropwise addition, the flask contents were stirred for 2 hours, and PERBUTYL O (0.3 g) was further added. The system was further stirred for 3 hours, bringing the reaction to completion. Subsequently, the solvent was driven off under reduced pressure (110°C, ≤ 1 kPa), giving the target copolymer. The number average molecular weight Mn was 21,630.

### [Comparative Example 4]

Isopropyl alcohol (23.3 g) was added to a separable flask and was heated at 100°C. Stearyl methacrylate (15.8 g), 2-hydroxyethyl methacrylate (17.5 g), isopropyl alcohol (16.6 g), and PERBUTYL O (manufactured by NOF Corporation) (2.0 g) were mixed together, and the mixture was added dropwise to the separable flask through a dropping funnel over a period of 1 hour. After the dropwise addition, the flask contents were stirred for 2 hours, and PERBUTYL O (0.3 g) was further added. The system was further stirred for 3 hours, bringing the reaction to completion. Subsequently, the solvent was driven off under reduced pressure (110°C, ≤ 1 kPa), giving the target copolymer. The number average molecular weight Mn was 15,617.

### [Comparative Example 5]

Isopropyl alcohol (23.3 g) was added to a separable flask and was heated at 100°C. Stearyl methacrylate (8.3 g), 2-hydroxyethyl methacrylate (25.0 g), isopropyl alcohol (16.6 g), and PERBUTYL O (manufactured by NOF Corporation) (2.0 g) were mixed together, and the mixture was added dropwise to the separable flask through a dropping funnel over a period of 1 hour. After the dropwise addition, the flask contents were stirred for 2 hours, and PERBUTYL O (0.3 g) was further added. The system was further stirred for 3 hours, bringing the reaction to completion. Subsequently, the solvent was driven off under reduced pressure (110°C, ≤ 1 kPa), giving the target copolymer. The number average molecular weight Mn was 18,937.

### [Comparative Example 6]

Isopropyl alcohol (23.3 g) was added to a separable flask and was heated at 100°C. Stearyl methacrylate (11.6 g), 2-hydroxyethyl methacrylate (8.4 g), the silicone macromer (13.3 g) represented by general formula (6), isopropyl alcohol (16.6 g), and PERBUTYL O (manufactured by NOF Corporation) (2.0 g) were mixed together, and the mixture was added dropwise to the separable flask through a dropping funnel over a period of 1 hour. After the dropwise addition, the flask contents were stirred for 2 hours, and PERBUTYL O (0.3 g) was further added. The system was further stirred for 3 hours, bringing the reaction to completion. Subsequently, the solvent was driven off under reduced pressure (110°C, ≤ 1 kPa), giving the target copolymer. The number average molecular weight Mn was 20,670.

### [Comparative Example 7]

Isopropyl alcohol (23.3 g) was added to a separable flask and was heated at 100°C. Stearyl methacrylate (19.0 g), 2-hydroxyethyl methacrylate (1.0 g), the silicone macromer (13.3 g) represented by general formula (6), isopropyl alcohol (16.6 g), and PERBUTYL O (manufactured by NOF Corporation) (2.0 g) were mixed together, and the mixture was added dropwise to the separable flask through a dropping funnel over a period of 1 hour. After the dropwise addition, the flask contents were stirred for 2 hours, and PERBUTYL O (0.3 g) was further added. The system was further stirred for 3 hours, bringing the reaction to completion. Subsequently, the solvent was driven off under reduced pressure (110°C, ≤ 1 kPa), giving the target copolymer. The number average molecular weight Mn was 22,240.

### [Evaluation of gelling ability]

Two grams of each of the copolymers obtained in Examples and Comparative Examples was dissolved into 8 g of various oils (Isododecane (INCI), Isostearyl Alcohol (INCI), decamethylcyclopentasiloxane (D5) (INCI: Cyclopentasiloxane)). The mixtures were brought to room temperature (25°C), thereby forming gels. The gelling ability was evaluated. The evaluation results were rated as "⊚" when the gelling agent formed a gel with high heat resistance (remained gelled even at 50°C), "○" when the gelling agent formed a gel, "△" when the gelling agent increased the viscosity, "×" when the gelling agent formed a solution, and "insoluble" when the gelling agent did not dissolve into the oil. The ratings "○" and above are acceptable. From the comparison of Examples with Comparative Examples, the gelling agents of Examples successfully allowed both of the hydrophobic oil and the higher alcohol to be gelled. Furthermore, the gelling agent of Example 5 was capable of gelation of the silicone-based oil and also showed thickening effects for the other oils.

**[Table 1]**

| Composition (g) | | Example 1 | Example 2 | Example 3 | Example 4 |
|---|---|---|---|---|---|
| (a) | AM1 | 31.6 | | | |
| | AM2 | | 31.6 | 30.0 | 28.3 |
| (a)' | Stearyl methacrylate | | | | |
| (b) | 2-Hydroxyethyl methacrylate | 1.7 | 1.7 | 3.3 | 5.0 |
| Number average molecular weight Mn | | 29,595 | 29,578 | 34,493 | 37,010 |
| (b)/(a) | | 0.054 | 0.054 | 0.11 | 0.18 |
| (b)/(a)' | | | | | |
| Gelling ability | Isododecane | ○ | ○ | ○ | ⊚ |
| | Isostearyl alcohol | ○ | ○ | ○ | ○ |
| | D5 | Insoluble | Insoluble | Insoluble | Insoluble |

**[Table 2]**

| Composition (g) | | Comp. Example 1 | Comp. Example 2 | Comp. Example 3 | Comp. Example 4 | Comp. Example 5 |
|---|---|---|---|---|---|---|
| (a) | AM1 | 26.6 | | | | |
| | AM2 | | 26.6 | | | |
| (a)' | Stearyl methacrylate | | | 30.0 | 15.8 | 11.4 |
| (b) | 2-Hydroxyethyl methacrylate | 6.7 | 6.7 | 3.3 | 17.5 | 13.6 |
| Number average molecular weight Mn | | 30,128 | 35,280 | 21,630 | 15,617 | 18,937 |
| (b)/(a) | | 0.25 | 0.25 | | | |
| (b)/(a)' | | | | 0.11 | 1.1 | 1.2 |
| Gelling ability | Isododecane | ○ | ○ | × | ○ | Insoluble |
| | Isostearyl alcohol | Δ | Δ | × | Δ | Δ |
| | D5 | Insoluble | Insoluble | Insoluble | Insoluble | Insoluble |

**[Table 3]**

| Composition (g) | | Example 5 | Comp. Example 6 | Comp. Example 7 |
|---|---|---|---|---|
| (a) | AM2 | 19.0 | | |
| (a)' | Stearyl methacrylate | | 11.6 | 19.0 |
| (b) | 2-Hydroxyethyl methacrylate | 1.0 | 8.4 | 1.0 |
| (c) | Silicone macromer (6) | 13.3 | 13.3 | 13.3 |
| Number average molecular weight Mn | | 35,450 | 20,670 | 22,240 |
| (b)/(a) | | 0.053 | 0.72 | 0.05 |
| Silicone macromer (wt%) | | 40.0 | 40.0 | 40.0 |
| Gelling ability | Isododecane | ○ | × | × |
| | Isostearyl alcohol | ○ | × | × |
| | D5 | ○ | Δ | × |

Formulation Examples of oil-based cosmetics and water-in-oil emulsion type cosmetics formulated with copolymers of the present invention are given below. The present invention is not limited by these Formulation Examples. The amounts given below indicate the amounts in which the compounded ingredients are included.

### [Formulation Example 1] Lipstick

| | (Ingredients) | Weight (%) |
|---|---|---|
| 1. | Polyethylene (INCI) | 14.0 |
| 2. | Microcrystalline Wax (INCI) | 4.0 |
| 3. | Polybutene (INCI) | 10.0 |
| 4. | Oil-gelling agent described in Example 4 | 15.0 |
| 5. | Cetyl octanoate (INCI: Cetyl Ethylhexanoate) | 20.0 |
| 6. | Triisostearin (INCI) | 37.0 |
| 7. | Pigment | q.s. |
| 8. | Preservative | q.s. |
| 9. | Fragrance | q.s. |
| | Total | 100.0 |

### (Method of Preparation)

A: Ingredients 5 and 6 were uniformly mixed.
B: Ingredients 1 to 4 were heated (90°C) and dissolved, then added to the mixture obtained in A, and rendered uniform.
C: Ingredients 7 to 9 were added to the mixture obtained in B at 80°C and rendered uniform.

The lipstick obtained in this way went on lightly without being oily or powdery, giving the lips a fresh feeling. Moreover, it had a good water resistance and water repellency, was long-lasting, and also had excellent stability.

### [Formulation Example 2] Oil-in-Water Cream

| | (Ingredients) | Weight (%) |
|---|---|---|
| 1. | Oil-gelling agent described in Example 1 | 8.0 |
| 2. | Crosslinked methyl phenyl polysiloxane (*1) ((dimethicone/phenyl vinyl dimethicone) crosspolymer (INCI: Diphenylsiloxy Phenyl Trimethicone)) | 2.0 |
| 3. | Isotridecyl Isononanoate (INCI) | 5.0 |
| 4. | DPG (INCI) | 7.0 |
| 5. | Glycerin (INCI) | 5.0 |
| 6. | Methylcellulose (INCI) (2% aqueous solution) (*2) | 7.0 |
| 7. | Polyacrylamide-based emulsifying agent (*3) (Polyacrylamide (INCI), (C13-14) Isoparaffin (INCI), Laureth-7 (IN CI), water) | 2.0 |
| 8. | Guanine (INCI) | 1.0 |
| 9. | Preservative | q.s. |
| 10. | Fragrance | q.s. |
| 11. | Purified water | 63.0 |
| | Total | 100.0 |

| | | |
|---|---|---|
| (*1) KSG-18, from Shin-Etsu Chemical Co., Ltd. (*2) Metolose SM-4000, from Shin-Etsu Chemical Co., Ltd. (*3) Sepigel 305, from SEPPIC | | |

### (Method of Preparation)

A: Ingredients 4 to 11 were mixed together.
B: Ingredients 1 to 3 were mixed together, the mixture obtained in A was added thereto, and the mixture was emulsified by stirring.

The cream obtained in this way had a fine texture and spread lightly without being sticky or oily, and moreover was moist and dewy, leaving the skin feeling fresh. This O/W cream was also confirmed to be very long-lasting and to have excellent stability, remaining unchanged with fluctuations in temperature and over time.

### [Formulation Example 3] W/O Cream Foundation

| | (Ingredients) | Weight (%) |
|---|---|---|
| 1. | Oil-gelling agent described in Example 4 | 4.0 |
| 2. | Polyether/Alkyl Co-Modified Branched Silicone (*1) (INCI: PEG-9/Lauryl Polydimethylsiloxyethyl Dimethicone Crosspolymer) | 1.0 |
| 3. | Triethylhexanoin (INCI) | 2.0 |
| 4. | Cetyl octanoate (INCI: Cetyl Ethylhexanoate) | 5.0 |
| 5. | Isotridecyl Isononanoate (INCI) | 9.0 |
| 6. | Hybrid silicone composite powder (*2) (INCI: (Vinyl Dimethicone/Methicone Silsesquioxane) Crosspolymer) | 3.0 |
| 7. | Polyglycerol co-modified branched silicone (*3) (INCI: Polyglyceryl-3-Disiloxane Dimethicone) | 0.6 |
| 8. | Polyglycerol co-modified branched silicone (*4) (INCI: Laurylpolyglyceryl-3 Polydimethylsiloxyethyl Dimethicone) | 0.3 |
| 9. | Alkyl/silicone treated pigment (*5) (INCI: Triethoxysilylethyl Polydimethylsiloxyethylhexyl Dimethicone) | 10.0 |
| 10. | BG (INCI: Butylene Glycol) | 5.0 |
| 11. | Sodium chloride | 0.5 |
| 12. | Sodium citrate | 0.2 |
| 13. | Preservative | q.s. |
| 14. | Fragrance | q.s. |
| 15. | Purified water | 59.4 |
| | Total | 100.0 |

| | | |
|---|---|---|
| (*1) KF-6038, from Shin-Etsu Chemical Co., Ltd. (*2) KSP-100, from Shin-Etsu Chemical Co., Ltd. (*3) KF-6100, from Shin-Etsu Chemical Co., Ltd. (*4) KF-6105, from Shin-Etsu Chemical Co., Ltd. (*5) KF-9909, from Shin-Etsu Chemical Co., Ltd. | | |

### (Method of Preparation)

A: Ingredients 1 to 6 were uniformly mixed.
B: Ingredients 7 to 10 were uniformly mixed.
C: Ingredients 11 to 13 and part of Ingredient 15 were mixed together and dissolved.
D: The mixture obtained in B was added to the balance of Ingredient 15 and rendered uniform.
E: The mixture obtained in C was added to the mixture obtained in A and emulsified.
F: The mixture obtained in D was added to the mixture obtained in E and emulsified. Last of all, Ingredient 14 was added and rendered uniform.

The W/O cream foundation obtained in this way was not sticky and spread easily. In addition, it had good pigment dispersibility, adhered well to the skin, and felt natural, forming a very clean film with a matte finish.

### [Formulation Example 4] Eye Color

| | (Ingredients) | Weight (%) |
|---|---|---|
| 1. | Glycol Distearate (INCI) | 12.0 |
| 2. | Oil-gelling agent described in Example 4 | 5.0 |
| 3. | Isotridecyl Isononanoate (INCI) | 35.0 |
| 4. | Candelilla wax (INCI: Euphorbia Cerifera (Candelilla) Wax) | 2.0 |
| 5. | Lecithin (INCI) | 0.2 |
| 6. | Hybrid silicone composite powder (*1) | 3.0 |
| | (INCI: (Vinyl Dimethicone/Methicone Silsesquioxane) Crosspolymer | r) |
| 7. | Alkyl/silicone treated pigment (*2) (Iron Oxides (INCI), Triethoxysilylethyl Polydimethylsiloxyethylhexyl Dimethicone (INC | q.s. |
| 8. | Mica-treated titanium oxide | balance |
| | Total | 100.0 |

| | | |
|---|---|---|
| (*1) KSP-100, from Shin-Etsu Chemical Co., Ltd. (*2) KTP-09, from Shin-Etsu Chemical Co., Ltd. | | |

A: Ingredients 2 and 3 were mixed together.
B: Ingredients 6 to 8 were mixed together
C: Ingredients 1, 4, and 5 were mixed together, the mixture obtained in A was added thereto, and the mixture was warmed.
D: The mixture obtained in C was added to the mixture obtained in B, and the mixture was poured into a container.

The eye color obtained in this way was not oily or sticky, spread lightly, and had a fresh feeling on use. In addition, it adhered well to the skin, felt natural, and was very long-lasting.

Cosmetics formulated with the oil-gelling agent of the present invention spread lightly and leave the skin feeling smooth and dry, providing the user with a feeling of freshness. Hence, by applying the cosmetic of the present invention, a smooth, soft emollient effect is imparted without the loss of a suitable degree of moisture transpiration, enabling a broad range of properties, from a natural luster to a matte feel, to be conferred. The cosmetics of the present invention thus have an excellent usability and also a good stability over time.

### INDUSTRIAL APPLICABILITY

The cosmetics of the present invention are comfortable to use, have a good stability over time, and are thus highly useful in practice. Moreover, the composition of the present invention, being a starting ingredient for cosmetics in practical use, has a high industrial utility.

## Claims

1. An oil-gelling agent comprising a copolymer comprising constituent units from:
an amphiphilic (meth)acrylic monomer (a) represented by general formula (1) below: 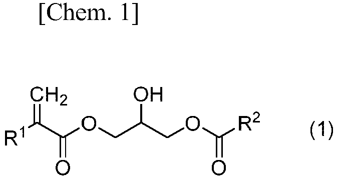 wherein R¹ is a hydrogen atom or a methyl group and R² is a linear or branched C₁₋₂₁ alkyl group; and
a hydrophilic (meth)acrylic monomer (b) represented by general formula (2) below: 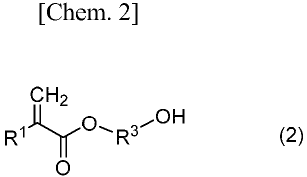 wherein R¹ is a hydrogen atom or a methyl group and R³ is a linear or branched C₂₋₁₀ alkylene group,
the mass ratio (b)/(a) of (b) to (a) being 0 < [(b)/(a)] ≤ 0.2.

2. The oil-gelling agent according to claim 1, further comprising constituent units from 35 to 50 wt% of a monomer (c) based on all the monomers, the monomer (c) being a silicone macromer represented by general formula (3) below: 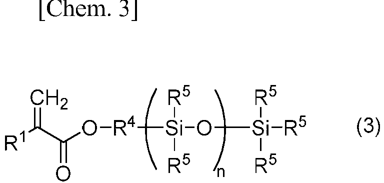 wherein R¹ is a hydrogen atom or a methyl group, R⁴ is a divalent organic group, R⁵ independently at each occurrence is a group selected from a hydrogen atom, a C₁₋₁₀ alkyl group, and a C₆₋₂₂ aryl group, and n is 1 to 100.

3. A cosmetic comprising the oil-gelling agent described in claim 1 or 2.

## Patentansprüche

1. Öl-Geliermittel, das ein Copolymer umfasst, das Grundeinheiten von Folgendem umfasst:
einem amphiphilen (Meth)acrylat-Monomer (a), das durch die nachfolgende allgemeine Formel (1) dargestellt ist: 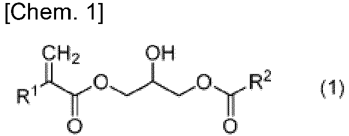 worin R¹ ein Wasserstoffatom oder eine Methylgruppe ist und R² eine unverzweigte oder verzweigte C₁₋₂₁-Alkylgruppe ist, und
einem hydrophilen (Meth)acrylat-Monomer (b), das durch die nachfolgende allgemeine Formel (2) dargestellt ist: 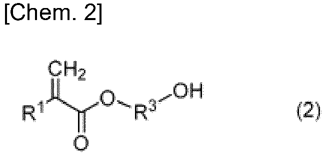 worin R¹ ein Wasserstoffatom oder eine Methylgruppe ist und R³ eine unverzweigte oder verzweigte C₂₋₁₀-Alkylengruppe ist;
wobei das Massenverhältnis (b)/(a) von (b) zu (a) 0 < [(b)/(a)] ≤ 0,2 beträgt.

2. Öl-Geliermittel nach Anspruch 1, der weiters 35 bis 50 Gew.-% Grundeinheiten eines Monomers (c), bezogen auf alle Monomere, umfasst, wobei das Monomer (c) ein Silikon-Makromer ist, das durch die nachfolgende allgemeine Formel (3) dargestellt ist: 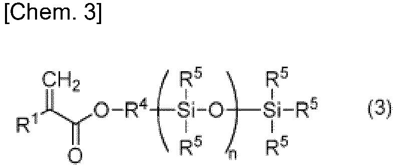 worin R¹ ein Wasserstoffatom oder eine Methylgruppe ist, R⁴ eine zweiwertige organische Gruppe ist, die R⁵ jeweils unabhängig bei jedem Vorkommen eine Gruppe sind, die aus einem Wasserstoffatom, einer C₁₋₁₀-Alkylgruppe und einer C₆₋₂₂-Arylgruppe ausgewählt ist, und n = 1 bis 100 ist.

3. Kosmetikum, das ein Öl-Geliermittel wie in Anspruch 1 oder 2 beschrieben umfasst.

## Revendications

1. Agent de gélification d'huile comprenant un copolymère comprenant des unités constitutives provenant de :
un monomère (méth)acrylique amphiphile (a) représenté par la formule générale (1) ci-dessous : 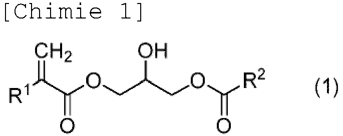
où R¹ est un atome d'hydrogène ou un groupe méthyle et R² est un groupe alkyle en C₁₋₂₁ linéaire ou ramifié ; et
un monomère (méth)acrylique hydrophile (b) représenté par la formule générale (2) ci-dessous : 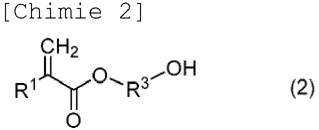
où R¹ est un atome d'hydrogène ou un groupe méthyle et R³ est un groupe alkylène en C₂₋₁₀ linéaire ou ramifié,
le rapport de masse (b)/(a) entre (b) et (a) étant de 0 < [(b)/(a)] ≤ 0,2.

2. Agent de gélification d'huile selon la revendication 1, comprenant en outre des unités constitutives de 35 à 50 % d'un monomère (c) sur la base de tous les monomères, le monomère (c) étant un macromère de silicone représenté par la formule générale (3) ci-dessous : 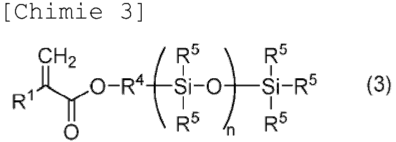 où R¹ est un atome d'hydrogène ou un groupe méthyle, R⁴ un groupe organique divalent, R⁵, indépendamment à chaque occurrence, est un groupe sélectionné parmi un atome d'hydrogène, un groupe alkyle en C₁₋₁₀ et un groupe aryl en C_{6- 22}, et n est compris entre 1 et 100.

3. Produit cosmétique comprenant l'agent de gélification d'huile décrit dans la revendication 1 ou 2.
